# EUROPEAN PATENT SPECIFICATION

(11) **EP 4 333 966 B1**
(45) Date of publication and mention of the grant of the patent: **13.08.2025**
(21) Application number: 22741576.7
(22) Date of filing: 27.06.2022
(51) Int. Cl.: A61N 1/04, A61N 1/36, A61N 1/40

(54) **SYSTEM FOR DELIVERING TUMOR TREATING FIELDS (TTFIELDS) AND MEASURING IMPEDANCE**
SYSTEM ZUR ABGABE VON TUMORBEHANDLUNGSFELDERN (TTFIELDS) UND IMPEDANZMESSUNG
SYSTEME PERMETTANT DE FOURNIR DES CHAMPS DE TRAITEMENT DES TUMEURS (TTFIELDS) ET DE MESURER L'IMPEDANCE

(30) Priority: 30.06.2021 US 202163216763 P
(43) Date of publication of application: 13.03.2024
(73) Proprietor: Novocure GmbH, 6340 Baar (CH)
(72) Inventor: WASSERMAN, Yoram, 31905 Haifa (IL); WEINBERG, Uri, 31905 Haifa (IL); OBUCHOVSKY, Stas, 31905 Haifa (IL); KUPLENNIK, Nataliya, 31905 Haifa (IL); SHAPIRO, David, 31905 Haifa (IL); SHTOTLAND, Michael, 31905 Haifa (IL); KIRILLOV, Sergei, 31905 Haifa (IL)
(74) Representative: Boden, Keith McMurray
(86) International application number: PCT/IB2022/055955
(87) International publication number: WO 2023/275718

(56) References cited:
- WO-A1-2021/069966
- WO-A1-2021/102120
- US-A1- 2017 095 660
- US-A1- 2018 050 200
- US-A1- 2019 083 782
- US-A1- 2019 160 292
- US-A1- 2020 155 835

## Description

### BACKGROUND

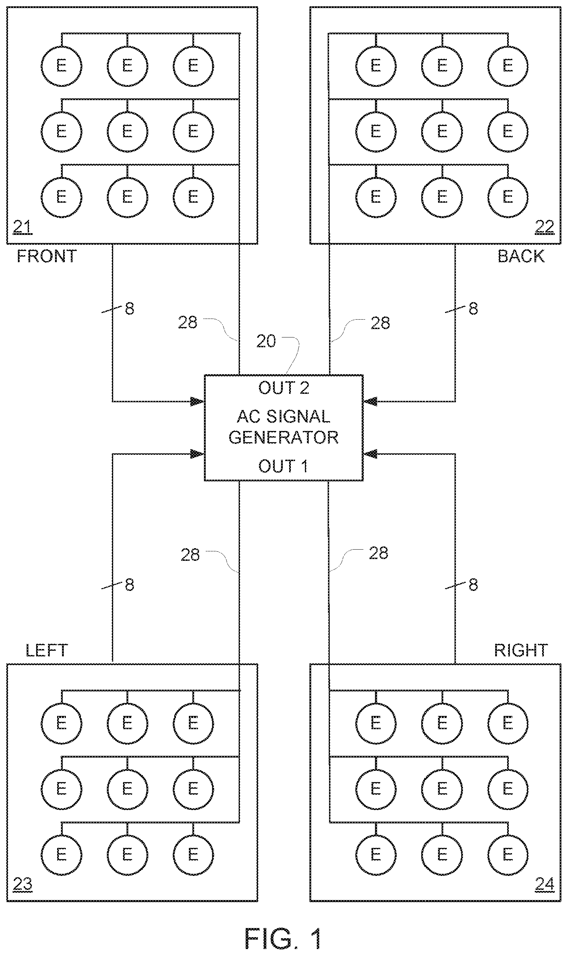

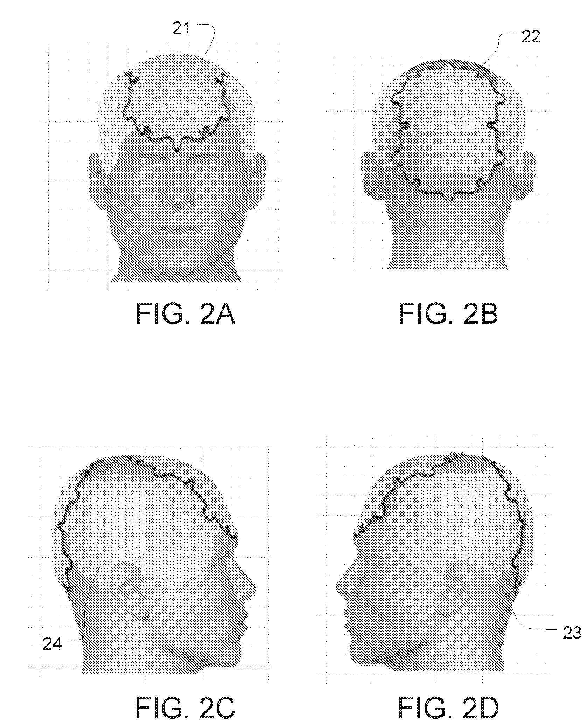

TTFields therapy is a proven approach for treating tumors. FIG. 1 is a schematic representation of the prior art Optune^{®} system for delivering TTFields. The TTFields are delivered to patients via four transducer arrays 21-24 that are placed on the patient's skin in close proximity to a tumor (e.g., as depicted in FIGS. 2A-2D for a person with glioblastoma). The transducer arrays 21-24 are arranged in two pairs, and each transducer array is connected via a multi-wire cable to an AC signal generator 20. The AC signal generator (a) sends an AC current through one pair of arrays 21, 22 during a first period of time, which induces an electric field with a first direction through the tumor; then (b) sends an AC current through the other pair of arrays 23, 24 during a second period of time, which induces an electric field with a second direction through the tumor; then repeats steps (a) and (b) for the duration of the treatment.

Each transducer array 21-24 is configured as a set of capacitively coupled electrode elements E (e.g., a set of 9 electrode elements, each of which is about 2 cm in diameter) that are interconnected via a flex circuit. Each electrode element includes an electrically conductive substrate with a dielectric layer (e.g., a layer of ceramic material with a high dielectric constant) disposed thereon. Each electrode element is sandwiched between a layer of an electrically conductive medical gel and an adhesive tape. When placing the arrays on the patient, the medical gel conforms to the contours of the patient's skin and ensures good electric contact of the device with the body. The adhesive tape holds the entire array in place on the patient as the patient goes about their daily activities. The transducer arrays are used to deliver long-term TTFields therapy (e.g., 15 hours per day over the course of at least one month).

US-A-2020/0155835 discloses transducer arrays for delivering tumor treating fields (TTFields) with selectively-addressable electrode elements.

US-A-2019/0160292 discloses implantable medical devices which incorporate a tissue conduction communication (TCC) transmitter, with the TCC transmitter being configured to generate at least one beacon signal during a wake-up mode and transmit data signals during a transmission mode.

WO-A-2021/102120 discloses apparatus for providing tumor treating fields (TTFields) to a target site within a patient which utilize an implanatble device proximate the target site, where the direction of the TTFields is changed by switching the polarities of the electrodes inducing the TTFields.

### SUMMARY OF THE INVENTION

One aspect of the invention is directed to an apparatus for applying alternating current between at least four first electrode elements positioned on a first side of a body and at least four second electrode elements positioned on a second side of the body according to claim 1.

Embodiments of the invention are disclosed in the dependent claims.

### BRIEF DESCRIPTION OF THE DRAWINGS

FIG. 1 is a schematic representation of the prior art Optune^{®} system for delivering TTFields.
FIGS. 2A-2D depict the positioning of transducer arrays on a person's head for treating a brain tumor.
FIG. 3 depicts a first embodiment of a transducer array that provides an individual conductor for each individual electrode element.
FIGS. 4A, 4B, and 4C are block diagrams of three embodiments, each of which uses four copies of the FIG. 3 transducer array to apply TTFields to a subject.
FIG. 5 is a flowchart of one example for detecting a condition of a region of skin on a subject's body using electrode elements positioned on a single side of the subject's body.
FIG. 6 is a flowchart of one example for detecting electrode element integrity using electrode elements positioned on a single side of a body.
FIG. 7 is a flowchart of one example for detecting a condition of a region of skin on a subject's body using electrode elements positioned on two sides of the subject's body.
FIG. 8 is a flowchart of one example for detecting electrode element integrity using electrode elements positioned on two sides of a body.
FIG. 9 is a schematic diagram of a circuit that is suitable for implementing a single switch or a switch in a bank of switches.

Various embodiments are described in detail below with reference to the accompanying drawings, wherein like reference numerals represent like elements.

### DESCRIPTION OF THE PREFERRED EMBODIMENTS

Although the FIG. 1 approach described above is very effective for delivering TTFields to a tumor, the effectiveness of the treatment will drop if good electrical contact between each of the electrode elements in the four transducer arrays 21-24 and the person's body is not maintained. This can happen, for example, if one or more of the electrode elements has a flaw, such as a defect in the dielectric layer of the electrode element or if the hydrogel beneath an electrode element dries out.

The embodiments described herein advantageously provide the ability to detect the condition of the transducer array electrode elements so that good electrical contact with the patient's body can be maintained to improve TTFields treatment.

Another source of treatment variance is the person's body and its tolerance for long-term treatment. For example, the transducer arrays are positioned on the person's body in order to target a tumor with TTFields, however the person's skin under the transducer arrays can experience changes (e.g., rash, irritation, etc.) as a result of the treatments, or other factors can impact a person's tolerance for the treatment (e.g., underlying skin conditions).

The embodiments described herein advantageously provide the ability to detect the condition of skin on the patient's body and, if necessary, to adjust the treatment characteristics so that the patient is able to better tolerate the treatment.

Some embodiments detect transducer array electrode conditions and/or patient skin conditions by receiving impedance measurements using the same electrodes that are used to deliver TTFields. Embodiments can sequentially apply the AC signal to pairs or groups of electrode elements to receive a plurality of impedance measurements. These impedance measurements can indicate the condition of participating electrode element(s) and/or the condition of the patient's skin that underlies the participating electrode element(s).

Embodiments can include a single system with at least two modes of operations, where a) in a first mode the system delivers TTFields treatment to a patient's body, and b) in a second mode the system measures impedance values that indicate the condition of participating electrode elements and/or the condition of the patient's skin. The same transducer array configuration can implement both modes of operation based on control signals issued by a controller. In an example implementation, at least two transducer arrays are positioned on different sides of a patient's body. In the first mode (e.g., TTFields treatment mode), an AC signal is applied to the positioned transducer arrays to deliver targeted TTFields treatment to the patient. However, the administration of the TTFields treatment may be improved by determining certain conditions, such as the condition of skin under the positioned arrays and/or the condition of the arrays' individual electrode elements. In the second mode, the electrodes of at least one positioned transducer array are used to measure impedance values indicative of the skin conditions and/or electrode conditions. The administration of the TTFields treatment (i.e., the first mode of operation) can be adjusted to optimize results based on the conditions detected in the second mode. For example, electrode integrity can be determined prior to use of a new transducer array, to ensure the integrity of electrodes from a previously used transducer array, and generally to ensure that good electrical contact is maintained for the TTFields treatment. In addition or alternatively, the skin condition can be determined to ensure that the patient's body is tolerating the long term application of TTFields treatment and/or to change characteristics of the treatment to ensure the patient's skin health. Based on detected conditions, treatment characteristics such as transducer array positioning, participating electrodes, AC signal characteristics, and other characteristics can be adjusted to optimize treatment. In some instances, electrode conditions and/or skin conditions may call for a pause of the TTFields treatments, for example to safeguard the health and safety of the patient.

Embodiments can measure the impedance values using a variety of techniques. For example, in a first technique at least one first electrode of a first polarity is positioned proximate to at least one second electrode of a second polarity such that the first and second electrodes are directly coupled (e.g., by the subject's body). An AC signal is then applied between the positioned first and second electrodes and the impedance is measured. In a second technique, at least one first electrode of a first polarity is positioned on a first side of a subject's body and at least one second electrode of a second polarity is positioned on a second side of the subject's body such that the first and second electrodes are capacitively coupled. An AC signal is then applied between the positioned first and second electrodes and the impedance is measured.

Some embodiments that measure impedance using the first technique or second technique have an individual conductor for each of the plurality of electrode elements in each of the transducer arrays. Unlike the prior art configuration, in which all of the electrode elements E in each of the transducer arrays 21-24 are wired in parallel, the individual conductor per electrode element makes it possible to independently switch the current on and off for any given individual electrode element in any one of the arrays. For example, in either the first technique or the second technique, embodiments can sequentially apply an AC signal between different pairs or groups of electrode elements, and this can be achieved by the ability to independently switch the current on and off to individual electrode elements.

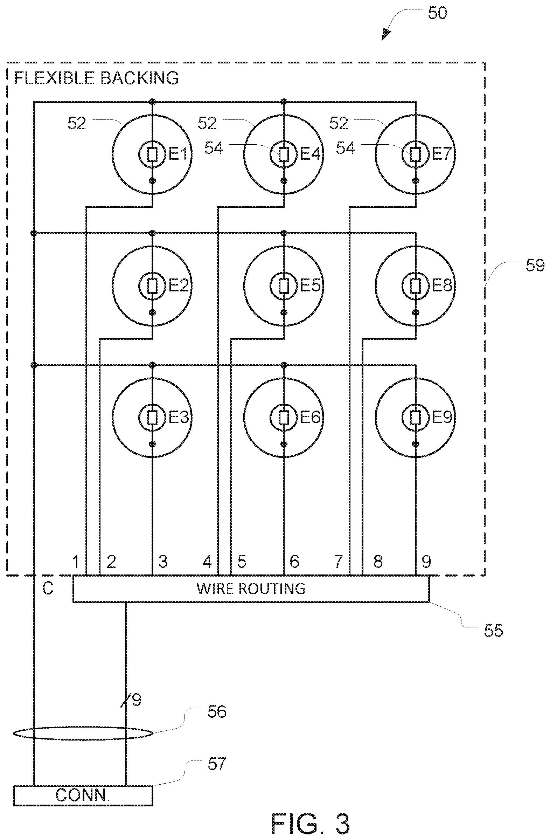

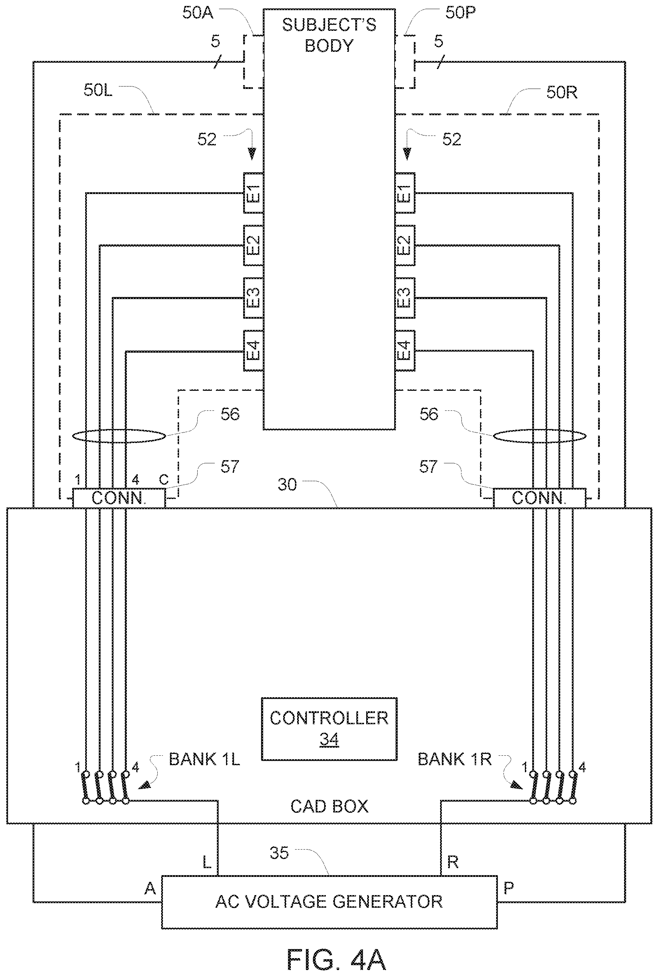

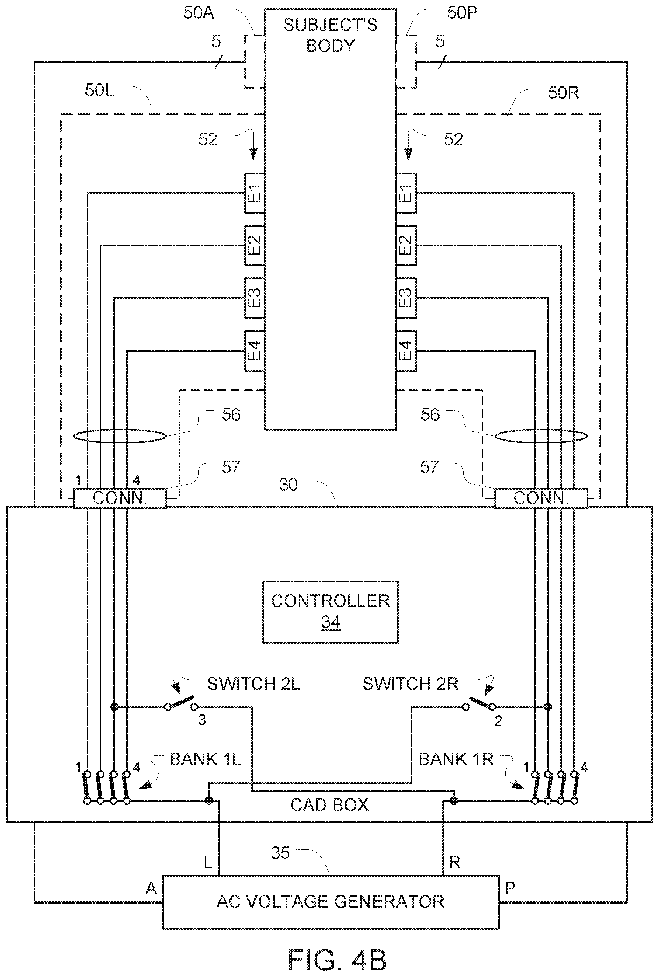

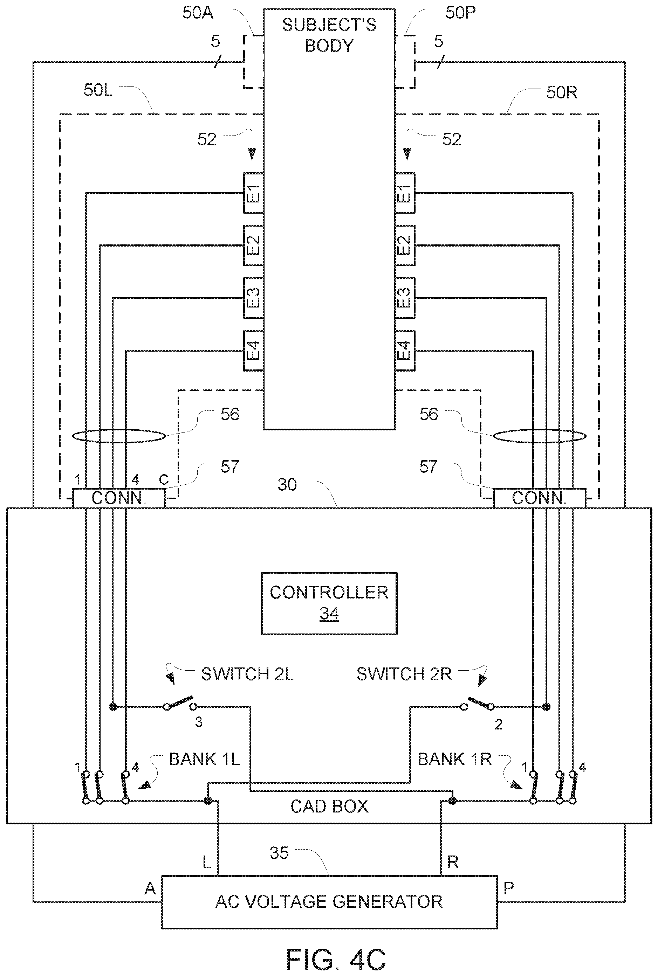

FIG. 3 depicts a first embodiment of a transducer array 50 that provides an individual conductor for each individual electrode element 52. As will be described below in connection with FIG. 4, four copies of the transducer array 50 are preferably used to administer TTFields treatment to a person's head (or other body part).

Each transducer array 50 includes a plurality of electrode elements 52, which are labeled E1-E9 for ease of reference in the FIG. 3 embodiment. Each of these electrode elements 52 has an electrically conductive substrate (e.g., a round metal substrate) with a dielectric layer disposed thereon. In some embodiments, each of these electrode elements 52 is a disc-shaped capacitively coupled electrode element (e.g., with a 2 cm diameter) that is similar to the prior art electrode elements used in the Optune^{®} system, and the dielectric layer comprises a thin layer of ceramic material with a high dielectric constant. However, unlike the Optune^{®} system and FIG. 1 (in which all the elements are wired in parallel), an individual conductor runs from each of the electrode elements 52 to the connector 57 in this FIG. 3 embodiment. These conductors are numbered 1-9 just above the "wire routing" block 55 (which funnels the individual conductors together into a single cable 56). In some embodiments, the electrical connection to each of the electrode elements 52 comprises one or more traces on a flex circuit and/or one or more conductive wires. In some embodiments, the dielectric layer comprises a polymer having a high dielectric constant (e.g., a dielectric constant greater than 10, preferably greater than 30).

In the embodiment depicted in FIG. 3, all of the capacitively coupled electrode elements 52 are held in place by a support structure 59. The support structure is configured to hold the electrode elements against the subject's body so that the dielectric layer of the electrode elements 52 faces the subject's body and can be positioned in contact with the subject's body. Optionally, this support structure may comprise a flexible backing 59 (e.g., a layer of foam material). Preferably, a layer of hydrogel or conductive adhesive is disposed between the dielectric layer of the electrode elements 52 and the subject's body when the transducer array 50 is placed against the subject's body. Construction of the support structure 59 may be implemented using any of a variety of conventional approaches that will be apparent to persons skilled in the relevant arts, including but not limited to self-adhesive fabric, foam, or plastic sheeting.

In some embodiments, each transducer array 50 also includes a plurality of thermistors 54 to sense the temperature of respective electrode elements 52. This may be accomplished, for example, by incorporating a hole or well in the center of each electrode element 52, and positioning a thermistor 54 in this hole or well. An example of suitable hardware and procedures that incorporate thermistors is described in U.S. Publication No. 2021/0196348, and suitable hardware and procedures that may be used to obtain temperature readings from the thermistors is described in U.S. Publication No. 2018/0050200.

Each transducer array 50 also has a connector 57 that is used to send electrical signals into and out of the transducer array 50. The connector 57 has a plurality of first pins and a second pin. In the illustrated embodiment, the number of first pins is the same as the number of electrode elements 52, and each of the first pins corresponds to a respective one of those electrode elements 52. And in the illustrated embodiment, there is only a single second pin, labeled C, which is provided for making temperature measurements. Note that as used herein, the term "pin" can refer to either a male or female pin of the connector 57.

Each transducer array 50 also has a plurality of first conductors, and the number of these first conductors will depend on the number of electrode elements 52. In the embodiment depicted in FIG. 3, which contains 9 electrode elements 52, these conductors are labeled 1-9. Each of these first conductors provides an electrically conductive path between (a) a respective one of the first pins in the connector 57, and (b) the conductive substrate of a respective one of the electrode elements 52 (E1-E9). Note that each of these first conductors may optionally be implemented using a plurality of segments of wire and/or a plurality of traces on a flex circuit.

Because the connector 57 has an individual first pin that corresponds to each of the individual electrode elements 52, and because an electrically conductive path exists between each of the first pins and a respective one of the electrode elements 52, the system that mates with the connector 57 can selectively energize or not energize each of the electrode elements 52 individually by either applying or not applying a signal to the respective first pin on the connector 57. Embodiments use transducer arrays 50 positioned on different sides of a patient's body to induce an electric field through the patient's body. For example, in a first mode of operation the transducer arrays can deliver TTFields treatment (e.g., using a target frequency between 100 and 500 kHz). In a second mode of operation the transducer arrays can induce an electric field through the patient's body using a lower frequency (e.g., below 20 kHz), and impedance measurements can be taken to detect a) conditions of the transducer arrays' electrode elements and/or b) conditions of the patient's skin.

FIG. 4A is a block diagram of a system that uses four copies of the transducer array 50 (described above in connection with FIG. 3) to a) apply TTFields to a subject; and b) take impedance measurements. In FIG. 4A, these four copies are labeled 50A, 50P, 50L, and 50R, where A, P, L, and R stand for anterior, posterior, left, and right, respectively. The lower portion of FIG. 4A depicts an AC voltage generator 35 and a "CAD box" 30 as separate blocks, the latter of which includes a controller 34 and banks of switches 1L and 1R. In some embodiments, the components in those two blocks 35, 30 may be physically divided into two separate housings. But in alternative embodiments, the components in those two blocks 35, 30 are combined into a single housing.

For clarity, the left and right channels are depicted in FIG. 4A. But the remaining channels (i.e., the anterior and posterior channels) operate in the same way as the left and right channels, respectively. Additionally, each of the transducer arrays 50 in FIG. 4A is depicted with only 4 electrode elements 52 for clarity. But it is expected that practical systems will have a larger number (e.g., between 9 and 30) of electrode elements, and will also have a larger number of certain other components (e.g., switches, conductors, etc.), depending on the number of electrode elements 52 that are actually used in each of the transducer arrays 50.

The FIG. 4A system can measure impedances to AC signals applied between pairs or among groups of electrode elements 52. For example, controller 34 can apply AC signals to any of transducer array 50L's electrode elements 52 by controlling the electronically-controlled switches in switch bank 1L (which may be implemented using bidirectional analog switches) to energize one or more of the electrode elements in turn. Similarly, controller 34 can apply AC signals to any of transducer array 50R's electrode elements 52 by controlling the electronically-controlled switches in switch bank 1R to energize one or more of the electrode elements in turn. In some embodiments, controller 34 can apply an AC signal to pairs or groups of electrode elements 52 positioned on two sides of the subject's body, such as by issuing control signals that electronically control switch banks 1L and 1R. Corresponding banks of switches (not shown) are also provided for the other channels 50A, 50P, and a similar approach is used in those channels as well.

Embodiments of the system of FIG. 4A implement multiple modes of operation: a) in the first mode of operation, a first polarity of an AC signal is applied to a majority of electrodes positioned at a first side of the subject's body (e.g., right or anterior) and a second polarity of the AC signal is applied to a majority of electrodes positioned at a second side of the subject's body (e.g., left or posterior); and b) in the second mode of operation, a first polarity of an AC signal is applied to at least one electrode positioned at a first side of the subject's body (e.g., right or anterior) and a second polarity of the AC signal is applied to at least one electrode positioned at a second side of the subject's body (e.g., left or posterior). In the first mode of operation the electrode elements can deliver TTFields treatment (e.g., using a target frequency between 100 and 500 kHz) while in the second mode of operation the electrode elements can induce an electric field through the patient's body using a lower frequency (e.g., below 20 kHz) such that impedance measurements can be taken. The impedances measurements are used to detect a) conditions of the transducer arrays' electrode elements and/or b) conditions of the patient's skin. The administration of the TTFields treatment (i.e., the first mode of operation) can be adjusted to optimize results given the detected conditions.

In both the first mode of operation (e.g., TTFields treatment mode) and the second mode of operation (e.g., impedance measurement mode), an electric field can be induced between the electrode elements positioned on the first side and the second side of the subject's body, however in the first mode the AC signal has a frequency range between 100 and 500 kHz (e.g., TTFields are administered) and in the second mode, the AC signal has a frequency below 20 kHz. The frequency range for the first mode of operation induces TTFields in the subject's body, while the frequency for the second mode of operation is selected so that impedance can be effectively measured. Using the lower frequency for the second mode of operation improves the quality of the electrode element integrity and/or skin condition measurements because it increases the impedances of capacitive portions of the circuit without increasing the impedances of any resistive portions of the circuit. It therefore pulls the desired signal (i.e., the contribution from the capacitive elements) up out of the background noise (i.e., the contribution from the resistive elements).

In some embodiments, during the second mode of operation, controller 34 can issue control signals that change the state of switch banks 1L and 1R to sequentially apply an AC signal between different pairs and/or among different groups of left and right electrode elements 52.

One approach for obtaining an impedance measurement that corresponds to each of the electrode elements in the transducer arrays 50L and 50R is as follows: (a) close all switches in bank 1L, close only switch 1 in bank 1R, and take one impedance measurement; (b) leave all the switches in bank 1L closed, open switch 1 in bank 1R, close switch 2 in bank 1R, then take another impedance measurement; (c) continue this process by stepping through each of the switches in bank 1R individually until an impedance measurement is obtained for each of the electrode elements in transducer array 50R; (d) close all switches in bank 1R, close only switch 1 in bank 1L, and take one impedance measurement; (e) leave all the switches in bank 1R closed, open switch 1 in bank 1L, close switch 2 in bank 1L, then take another impedance measurement; and (f) continue this process by stepping through each of the switches in bank 1L individually until an impedance measurement is obtained for each of the electrode elements in transducer array 50L. Each individual impedance measurement (for any given set of switch settings) may be made, for example, by dividing the output voltage of the AC voltage generator 35 (which can be either known in advance or measured each time) by the current that the AC voltage generator 35 is delivering, which will be measured in real time (using any conventional approach) for that particular set of switch settings.

Embodiments of the FIG. 4B system can implement a TTFields treatment mode of operation and an impedance measurement mode of operation using the same electrode elements, where, in the impedance measurement mode, impedance measurements are taken using a single transducer array 50 positioned on a single side of a patient's body. Transducer array(s) 50 are used to a) in a first mode of operation (e.g., TTFields treatment mode), induce an electric field and deliver TTFields treatment, and b) in a second mode of operation (e.g., impedance measurement mode), measure impedance to current between two or more local electrodes on a single transducer array. For example, in the second mode an AC signal can be applied between two or more local electrodes on a single transducer array 50, and impedance measurements can be taken. The impedance measurements can be indicative of a) conditions of the transducer array's electrode elements and/or b) conditions of the patient's skin.

The FIG. 4B system can apply TTFields to a subject and measure impedances to AC signals applied to pairs or among groups of electrode elements 52 in a manner similar to the FIG. 4A system, however the FIG. 4B system additionally includes switch(es) 2L and switch(es) 2R. In some embodiments, switch 2L and/or switch 2R can be controlled by controller 34 so that an AC signal is applied between pairs or among groups of electrode elements on a single side of the subject's body. For example, generally the system of FIG. 4A applies a first polarity of the AC signal to electrode elements positioned on a first side of the subject's body (e.g., right or anterior) and a second polarity of the AC signal to electrode elements positioned on a second side of the subject's body (e.g., left or posterior). This system configuration can induce an electric field through the subject's body.

The system of FIG. 4B includes switch bank 1L and at least one switch 2L, where switch bank 1L can apply a first polarity of an AC signal to one or multiple left side electrode elements 52 while switch 2L can apply a second polarity of the AC signal to at least one left side electrode element 52. Because transducer 50L is positioned so that left side electrode elements 52 are coupled by the subject's body, direct current flows between a pair or among a group of left side electrode elements 52 when the state of switch bank 1L and switch 2L causes both first and second polarities of the AC signal to be applied to left side electrode elements 52.

Embodiments of the system of FIG. 4B implement multiple modes of operation: a) in the first mode of operation (which is a TTFields treatment mode), a first polarity of an AC signal is applied to a majority of electrodes positioned at a first side of the subject's body (e.g., right or anterior) and a second polarity of the AC signal is applied to a majority of electrodes positioned at a second side of the subject's body (e.g., left or posterior). In the second mode of operation (which is an impedance measurement mode), a first polarity of an AC signal is applied to at least one electrode element positioned on a first side of the subject's body and a second polarity of the AC signal is applied to at least one electrode element positioned on the first side (i.e., the same side) of the subject's body.

In the illustrated embodiment of FIG. 4B, E3 of the left electrode elements 52 has a switchable polarity because this electrode element corresponds to two switches, one switch from switch bank 1L (used to apply a first polarity of an AC signal) and switch 2L (used to apply a second polarity of an AC signal). For example, in the first mode of operation, a first polarity of an applied AC signal (i.e., via a closed switch from switch bank 1L) can be applied to E3 while in the second mode of operation, a second polarity of an applied AC signal (i.e., via a closed switch 2L) can be applied to E3.

Consider left electrode elements 52 E1, E2, E3, and E4 depicted in FIG. 4B during the second mode of operation. When the second polarity of an AC signal is applied to E3 (i.e., switch 2L is closed), the first polarity of the AC signal can be applied to one of E1, E2, or E4 and current can flow via the subject's skin between the pair of electrode elements. Alternatively, when the second polarity of the AC signal is applied to E3, the first polarity of the AC signal can be applied to two or more of E1, E2, or E4, and current can flow among the group of electrode elements. In some embodiments, controller 34 can issue control signals that change the state of switch bank 1L and/or switch 2L to sequentially apply an AC signal between different pairs and/or among different groups of left electrode elements 52.

For example, sequentially applying AC signals to pairs of electrodes can include a first pair E3 and E1, a second pair E3 and E2, and a third pair E3 and E4. In another example, sequentially applying AC signals to groups of electrodes can include a first group E1, E2, and E3, a second group E2, E3, and E4, and a third group E1, E3, and E4. In both of these examples E3 is a consistent presence among the pairs or groups because E3 is illustrated as the switchable polarity electrode element in FIG. 4B. Other embodiments include multiple switchable polarity electrode elements and/or a higher number of electrode elements that can achieve many more possible pairs or groups of electrodes.

In some embodiments, impedance is measured during each sequential application of the AC signal to a pair or group of electrodes. For example, an AC signal can be applied to a first pair of electrode elements and impedance can be measured during the application; following the measurement, the AC signal can be halted and control signals from controller 34 can change the state of switch bank 1L and switch 2L; and then following the change of state, the AC signal can be applied to a second pair of electrodes and impedance can be measured, and so on.

While the illustrated embodiment depicts only one switch that routes the opposite polarity to a given electrode element 52, switch(es) 2L can include two, three, or more switches such that two, three, or more left electrode elements 52 have a switchable polarity. Switch(es) 2R and right electrode elements 52 can operate in a similar manner to switch(es) 2L and left electrode elements 52. Corresponding switches and banks of switches (not shown) are also provided for the other channels 50A, 50P, and a similar approach is used in those channels as well.

One approach for obtaining an impedance measurement that corresponds to each of the electrode elements in the transducer array 50L in the FIG. 4B embodiment is to: (a) close the switch 2L (e.g., corresponding to the switchable polarity electrode element E3), close only switch 1 in bank 1L, and take one impedance measurement; (b) leave switch 2L closed, open switch 1 in bank 1L, close switch 2 in bank 1L, then take another impedance measurement; (c) continue this process by stepping through each of the switches in bank 1L (except switch 3) individually until an impedance measurement is obtained for each of the electrode elements in transducer array 50L (except electrode element E3). A similar approach can be used to obtain an impedance measurement that corresponds to each of the electrode elements in the transducer array 50R. Each individual impedance measurement (for any given set of switch settings) may be made, for example, as described above for the FIG. 4A embodiment. After impedance measurements are obtained for each of the individual elements in the arrays 50L, 50R, those impedance measurements may be used, e.g., to modify TTFields treatment that is applied using those same arrays 50L, 50R.

Embodiments of the FIG. 4C system can implement a treatment mode of operation and an impedance measurement mode of operation similar to the system of FIG. 4B, however the impedance measurements are taken using a reverse polarity electrode element (rather than a switchable polarity electrode element). Transducer array(s) 50 are used to a) in a first mode of operation, induce an electric field and deliver TTFields treatment using most of the arrays' electrode elements, and b) in a second mode of operation, measure impedance to current between two or more local electrodes on a single transducer array, where at least one of the two electrodes is a reverse polarity electrode dedicated to the second mode of operation. For example, the transducer arrays 50 can be wired such that an applied AC signal applies a first polarity to most of the electrode elements of the array and applies a second polarity to at least one of the electrode elements of the array (i.e., the reverse polarity electrode element dedicated to the second mode of operation). In these embodiments, the electrode element dedicated to the second mode is selectively applied the second polarity such that a) the element is turned "OFF" when delivering TTFields treatment in the first mode, and b) the element is selectively turned "ON" when taking local impedance measurements in the second mode. For example, in the second mode an AC signal can be applied between the dedicated electrode element and at least one other electrode element (both on a single transducer array 50), and impedance measurements can be taken. The impedance measurements can be indicative of a) conditions of the transducer array's electrode elements and/or b) conditions of the patient's skin.

The FIG. 4C system can apply TTFields to a subject and measure impedances to AC signals applied to pairs or among groups of electrode elements 52 in a manner similar to the FIG. 4B system, however switch banks 1L and 1R in the FIG. 4C system have fewer connections to electrode elements 52. In FIG. 4B, at least one electrode element positioned on a given side of the subject's body has a switchable polarity because the electrode element corresponds to both one switch from switch bank 1L and switch 2L. In FIG. 4C, rather than an electrode element with a switchable polarity, a transducer 50 includes several electrode elements that can be applied a first polarity of an AC signal and at least one electrode element that can be connected to the opposite polarity of the AC signal.

In the embodiment illustrated in FIG. 4C, transducer 50L includes left electrode elements 52 E1, E2, E3, and E4, where E1, E2, and E4 are connected to AC generator 35 via switch bank 1L and E3 is connected to AC generator 35 via switch 2L. Based on control signals issued by controller 34, a first polarity of the AC signal can be applied to E1, E2, and/or E4 (via switch bank 1L) and a second polarity of the AC signal can be applied to E3 (via switch 2L). In the embodiment illustrated in FIG. 4B, left electrode element 52 E3 has a switchable polarity, while in the embodiment illustrated in FIG. 4C, left electrode element 52 E3 has an opposite polarity relative to the other left electrode elements 52 (but not a switchable polarity). Unlike the system of FIG. 4B where switchable polarity electrode element E3 is capable of delivering TTFields treatment by inducing an electric field in a manner similar to electrode elements E1, E2, and E4, in the system of FIG. 4C electrode element E3 does not have a switchable polarity and thus is not capable of delivering TTFields treatment by inducing an electric field in a manner similar to electrode elements E1, E2, and E4.

Embodiments of the system of FIG. 4C implement multiple modes of operation: a) in the first mode of operation (e.g., TTFields treatment mode), a first polarity of an AC signal is applied to a majority of electrodes positioned at a first side of the subject's body (e.g., right or anterior) and a second polarity of the AC signal is applied to a majority of electrodes positioned at a second side of the subject's body (e.g., left or posterior); b) and in the second mode of operation (e.g., impedance measurement mode), a first polarity of an AC signal is applied to at least one electrode element positioned on a first side of the subject's body and a second polarity of the AC signal is applied to at least one electrode element positioned on the first side of the subject's body. In the first mode of operation, TTFields can be induced between the electrode elements positioned on the first side and the second side of the subject's body. In the second mode of operation, current can flow between pairs or among groups of electrode elements positioned on the same side of the subject's body.

Consider left electrode elements 52 E1, E2, E3, and E4 depicted in FIG. 4C during the second mode of operation. When the second polarity of an AC signal is applied to E3 (i.e., switch 2L is closed), the first polarity of the AC signal can be applied to one of E1, E2, or E4 and current can flow between the pair of electrode elements. Alternatively, when the second polarity of the AC signal is applied to E3, the first polarity of the AC signal can be applied to two or more of E1, E2, or E4, and current can flow among the group of electrode elements. In some embodiments, controller 34 can issue control signals that change the state of switch bank 1L and/or switch 2L to sequentially apply an AC signal between different pairs and/or among different groups of left electrode elements 52.

For example, sequentially applying AC signals to pairs of electrodes can include a first pair E3 and E1, a second pair E3 and E2, and a third pair E3 and E4. In another example, sequentially applying AC signals to groups of electrodes can include a first group E1, E2, and E3, a second group E2, E3, and E4, and a third group E1, E3, and E4. In both of these examples E3 is a consistent presence among the pairs or groups because E3 is illustrated as the opposite polarity electrode element in FIG. 4C. Other embodiments include multiple opposite polarity electrode elements and/or a higher number of overall electrode elements that result in many more potential pairs or groups of electrode elements to which the AC signals can be sequentially applied.

In some embodiments, impedance is measured during each sequential application of the AC signal to a pair or group of electrodes. For example, an AC signal can be applied to a first pair of electrode elements and impedance can be measured during the application; following the measurement, the AC signal can be halted and control signals from controller 34 can change the state of switch bank 1L and switch 2L; and then following the change of state, the AC signal can be applied to a second pair of electrodes and impedance can be measured, and so on.

One approach for obtaining an impedance measurement that corresponds to each of the electrode elements in the transducer array 50L in the FIG. 4C embodiment is to: (a) close the switch 2L, close only switch 1 in bank 1L, and take one impedance measurement; (b) leave switch 2L closed, open switch 1 in bank 1L, close switch 2 in bank 1L, then take another impedance measurement; (c) continue this process by stepping through each of the switches in bank 1L individually until an impedance measurement is obtained for each of the electrode elements in transducer array 50L. A similar approach can be used to obtain an impedance measurement that corresponds to each of the electrode elements in the transducer array 50R. Each individual impedance measurement (for any given set of switch settings) may be made, for example, as described above for the FIG. 4A embodiment. After impedance measurements are obtained for each of the individual elements in the arrays 50L, 50R, those impedance measurements may be used, e.g., to modify TTFields treatment that is applied using those same arrays 50L, 50R.

While the illustrated embodiment of FIG. 4C depicts only one switch 2L, switch(es) 2L can include two, three, or more switches such that two, three, or more left electrode elements 52 have an opposite polarity. Switch(es) 2R and right electrode elements 52 can operate in a similar manner to switch(es) 2L and left electrode elements 52. Corresponding switches and banks of switches (not shown) are also provided for the other channels 50A, 50P, and a similar approach is used in those channels as well.

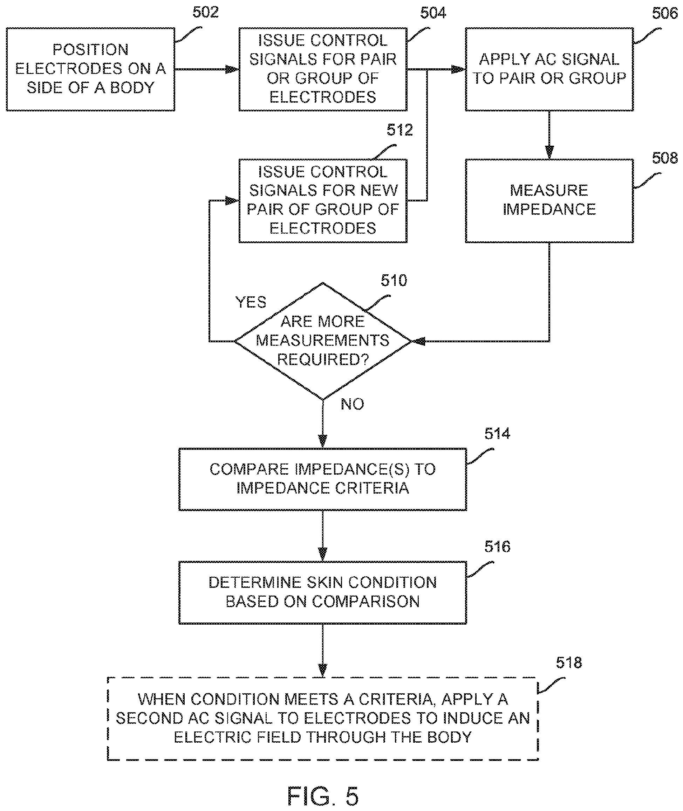

FIG. 5 is a flowchart of one example for detecting a condition of a region of skin on a subject's body using electrode elements positioned on a single side of the subject's body. For example, the transducer of FIG. 3 and the systems of FIG. 4B or FIG. 4C can be used to implement the flowchart of FIG. 5.

At step 502, electrode elements are positioned on a side of a body. For example, at least four electrode elements can be positioned on a subject's body so that each of the electrode elements is coupled to a respective region of skin on the same side of the body. In some embodiments, the electrode elements are part of an array (e.g., transducer array 50 of FIG. 3).

At step 504, control signals are issued that select a pair or group of the electrode elements. For example, a controller (e.g., controller 34 of FIGS. 4B and 4C) can issue control signals to configure switches (e.g., switch bank 1L and switch(es) 2L of FIGS. 4B and 4C) so that an electrically conductive path is formed from an AC signal generator to the selected pair or group of electrode elements.

At step 506 an AC signal is applied to the selected pair or group of electrode elements. The applied AC signal causes current to flow between the selected pair or among the selected group of electrode elements. At step 508, during the application of the AC signal to the selected pair or group, an impedance to the AC signal is measured and stored.

At step 510 it is determined whether more measurements are required. For example, it may be determined whether all pairs from a set of pairs of electrode elements has had an impedance measurement taken, whether all groups from a set of groups of electrode elements has had an impedance measurement taken, whether any additional pairs or groups of electrode elements is eligible for measurement, whether a threshold number of impedance measurements have been taken, or any other suitable criteria. When more measurements are required, the flowchart progresses along a loop to step 512. When more measurements are not required, the flowchart exits the loop and progresses to step 514.

At step 512, control signals are issued that select a new pair or group of the electrode elements. For example, a set of electrode element pairs or groups can be traversed by the loop of the FIG. 5 flowchart until each group or pair in the set has been selected for impedance measurement. From step 512 the flowchart loops back to steps 506 and 508, where the AC signal is applied to the new pair or group of electrode elements and during this application impedance measurements are taken and stored.

The flowchart of FIG. 5 loops through steps 506, 508, 510, and 512 until more measurements are not required at step 510. Once this loop break condition is achieved, the flowchart progresses to step 514, where the stored impedances are compared to an impedance criteria. For example, an impedance criteria can be a range of values or a threshold value, and the stored impedance values can be compared to this threshold or range. In some embodiments, the impedance criteria can include previously stored impedance values, such as historic values measured over time for multiple subjects or historic values measured over time for an individual subject. For example, a metric can be calculated based on these historic values. The measured and stored impedance values can be compared to these historic values or the metric calculated based on the historic values. In some embodiments, the comparing comprises comparing the measured impedances to a constant.

At step 516, a condition of the region of skin is determined based on the comparison. For example, it can be determined that the region of skin is in a first condition when the stored impedance values fall within an acceptable range of the criteria or meet a threshold of the criteria, and that the region of skin is in a second condition when the stored impedance values are outside of the acceptable range of the criteria or fail to meet (e.g., greater than or less than) the threshold of the criteria. In another embodiment, it can be determined that the region of skin is in a first condition when a delta between the stored impedance values and the historic impedance values or a metric based on the historic impedance values is less than or equal to a threshold delta, and that the region of skin is in a second condition when the delta between the stored impedance values and the historic impedance values or a metric based on the historic impedance values is greater than the threshold delta. In these examples, the first condition can indicate generally healthy skin and the second condition can indicate damaged skin.

Optionally, if the condition of the region of skin meets a criteria, the method can continue at step 518, where the electrode elements are used to induce an electric field through the subject's body. For example, when the impedance comparisons indicate that the region of skin is in the first condition, the criteria is met and a second AC signal (e.g., with a frequency between 100 and 500 kHz) is applied to the electrode elements to induce an electric field through the subject's body (e.g., to administer TTFields to the subject).

In some embodiments, based on the determined condition for the region of skin, the application of TTFields can be adjusted. For example, the current at any given electrode element positioned at the damaged skin can be reduced when applying the TTFields, or the TTFields therapy can be paused for a period of time so that the damaged skin can heal. Other suitable characteristics of the TTFields treatment can be adjusted based on the determined condition of the region of skin.

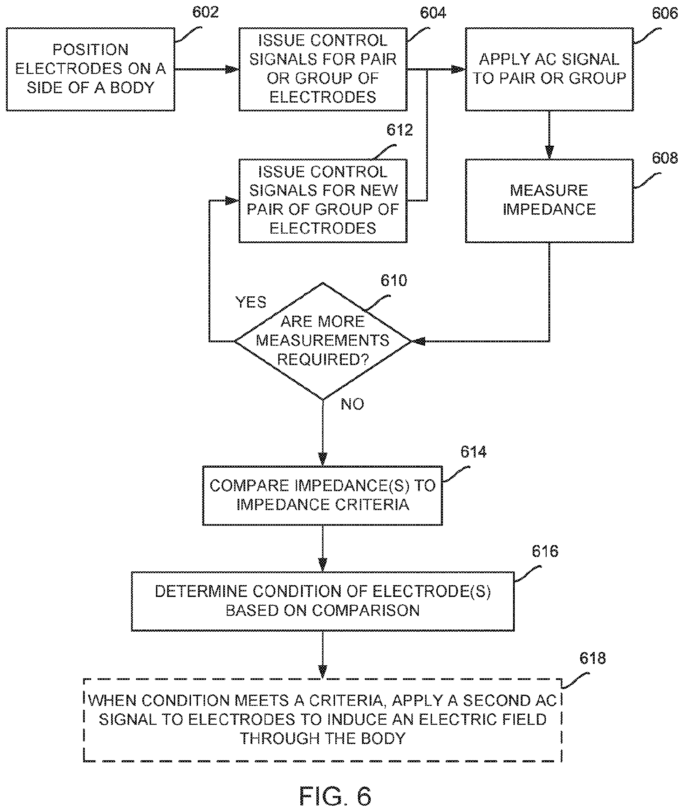

FIG. 6 is a flowchart of one example for detecting electrode element integrity using electrode elements positioned on a single side of a body. For example, the transducer of FIG. 3 and the systems of FIG. 4B or FIG. 4C can be used to implement the flowchart of FIG. 6.

At step 602, electrode elements are positioned on a side of a body. For example, at least four electrode elements can be positioned on a body. In some embodiments, the electrode elements are part of an array (e.g., transducer array 50 of FIG. 3).

At step 604, control signals are issued that select a pair or group of the electrode elements. For example, a controller (e.g., controller 34 of FIGS. 4B and 4C) issues control signals to configure switches (e.g., switch bank 1L and switch(es) 2L of FIGS. 4B and 4C) so that an electrically conductive path is formed from an AC signal generator to the selected pair or group of electrode elements.

At step 606 an AC signal is applied to the selected pair or group of electrode elements. The applied AC signal causes current to flow between the selected pair or among the selected group of electrode elements. At step 608, during the application of the AC signal to the selected pair or group, an impedance to the AC signal is measured and stored.

At step 610 it is determined whether more measurements are required. For example, it may be determined whether all pairs from a set of pairs of electrode elements has had an impedance measurement taken, whether all groups from a set of groups of electrode elements has had an impedance measurement taken, whether any additional pairs or groups of electrode elements is eligible for measurement, whether a threshold number of impedance measurements have been taken, or any other suitable criteria. When more measurements are required, the flowchart progresses along a loop to step 612. When more measurements are not required, the flowchart exits the loop and progresses to step 614.

At step 612, control signals are issued that select a new pair or group of the electrode elements. For example, a set of electrode element pairs or groups can be traversed by the loop of the FIG. 6 flowchart until each group or pair in the set has been selected for impedance measurement. From step 612 the flowchart loops back to steps 606 and 608, where the AC signal is applied to the new pair or group of electrode elements and during this application impedance measurements are taken and stored.

The flowchart of FIG. 6 loops through steps 606, 608, 610, and 612 until more measurements are not required at step 610. Once this loop break condition is achieved, the flowchart progresses to step 614, where a condition of one or more of the electrode elements is determined based on a comparison of the stored impedances with an impedance criteria. For example, an impedance criteria can be a range of values or a threshold value, and the stored impedance values can be compared to this threshold or range. Stored impedance values can be associated with the individual electrode elements that were selected (e.g., switched on) when the measurement was taken. The condition for these individual electrode elements can be determined by comparing their associated impedance value(s) to the impedance criteria. In some embodiments, the impedances can be compared to a range of values or a threshold value, previously stored impedance values (e.g., historic values measured over time for multiple electrode elements or historic values measured over time for an individual electrode element), or a metric calculated based on these historic values, In some embodiments, the comparing comprises comparing the measured impedances to a constant.

At step 616, a condition of electrode elements is determined based on the comparisons. For example, it can be determined that a given electrode element is in a first condition when the stored impedance values associated with the given electrode element fall within an acceptable range of the impedance criteria or meet a threshold of the impedance criteria, and that the given electrode element is in a second condition when the stored impedance values associated with the given electrode element are outside of the acceptable range of the impedance criteria or fail to meet (e.g., greater than or less than) the threshold of the impedance criteria. In another embodiment, it can be determined that the given electrode element is in a first condition when a delta between the stored impedance value(s) associated with the given electrode and historic impedance values or a metric based on the historic impedance values is less than or equal to a threshold delta, and that the given electrode element is in a second condition when the delta between the stored impedance value(s) associated with the given electrode and historic impedance values or a metric based on the historic impedance values is greater than the threshold delta.

In these examples, the first condition can indicate an operable condition for the given electrode element while the second condition can indicate a flawed condition for the given electrode element. Optionally, if the condition of the one or more electrode elements meets the criteria, the method can continue at step 618, where the electrode elements are used to induce an electric field through the body. For example, when the impedance comparisons indicate that the one or more electrode elements are in the first condition, the criteria is met and a second AC signal (e.g., with a frequency between 100 and 500 kHz) is applied to the electrode elements to induce an electric field through the body (e.g., to administer TTFields to the body). Based on the determined conditions for the electrode elements, the application of TTFields can be adjusted. For example, one or more electrode elements can be switched off so that these electrode elements do not participate in the application of TTFields. Other suitable characteristics of the TTFields treatment can be adjusted based on the determined condition of the electrode elements.

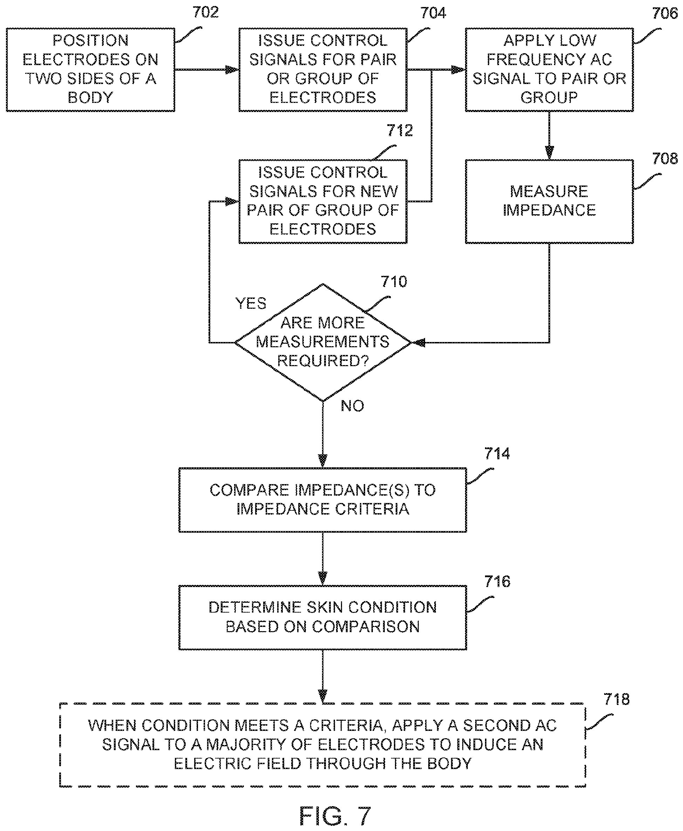

FIG. 7 is a flowchart of one example for detecting a condition of a region of skin on a subject's body using electrode elements positioned on two sides of the subject's body. For example, the transducer of FIG. 3 and the system of FIG. 4A can be used to implement the flowchart of FIG. 7.

At step 702, electrode elements are positioned on two sides of a body. For example, at least four first electrode elements are positioned on a first side of a subject's body and at least four second electrode elements are positioned on a second side of the subject's body. The first electrode elements are positioned so that each of the first electrode elements is coupled to a respective region of skin on the body. In some embodiments, the first electrode elements are part of an array (e.g., transducer array 50 of FIG. 3). In some embodiments, the second electrode elements are also part of an array.

At step 704, control signals are issued that select a subset of the first and second electrode elements. For example, a controller (e.g., controller 34 of FIG. 4A) issues control signals to configure switches (e.g., switch banks 1L and 1R of FIG. 4A) so that an electrically conductive path is formed from an AC signal generator to the selected pair or group of electrode elements. In embodiments, the subset comprises one or more of the first electrode elements and one or more of the second electrode elements.

At step 706, a low frequency AC signal is applied to the selected subset of electrode elements. For example, the AC signal can have a frequency below 20 kHz. The applied AC signal causes an electric field to be induced through the subject's body by the first electrode elements and second electrode elements that comprise the selected subset of electrode elements. At step 708, during the application of the AC signal to the selected subset, an impedance is measured and stored.

At step 710 it is determined whether more measurements are required. For example, it may be determined whether all subsets (e.g., pairs) from a set of subsets (pairs) of electrode elements has had an impedance measurement taken, whether any additional subsets of first electrode elements and second electrode elements is eligible for measurement, whether a threshold number of impedance measurements have been taken, or any other suitable criteria. When more measurements are required, the flowchart progresses along a loop to step 712. When more measurements are not required, the flowchart exits the loop and progresses to step 714.

At step 712, control signals are issued that select a new subset of the electrode elements. For example, a set of electrode element subsets, each comprising at least one first electrode element and at least one second electrode element, can be traversed by the loop of the FIG. 7 flowchart until each subset in the set has been selected for impedance measurement. From step 712 the flowchart loops back to steps 706 and 708, where the AC signal is applied to the new subset of electrode elements and during this application impedance measurements are taken and stored.

The flowchart of FIG. 7 loops through steps 706, 708, 710, and 712 until more measurements are not required at step 710. Once this loop break condition is achieved, the flowchart progresses to step 714, where a condition of the region of skin is determined based on a comparison of the stored impedances with an impedance criteria. For example, an impedance criteria can be a range of values or a threshold value, and the stored impedance values can be compared to this threshold or range. In some embodiments, the impedance criteria can include previously stored impedance values, such as historic values measured over time for multiple subjects or historic values measured over time for an individual subject. For example, a metric can be calculated based on these historic values. The measured and stored impedance values can be compared to these historic values or the metric calculated based on the historic values. In some embodiments, the comparing comprises comparing the measured impedances to a constant.

At step 716, a condition of the region of skin is determined based on the comparison. For example, it can be determined that the region of skin is in a first condition when the stored impedance values fall within an acceptable range of the criteria or meet a threshold of the criteria, and that the region of skin is in a second condition when the stored impedance values are outside of the acceptable range of the criteria or fail to meet (e.g., greater than or less than) the threshold of the criteria. In another embodiment, it can be determined that the region of skin is in a first condition when a delta between the stored impedance values and the historic impedance values or a metric based on the historic impedance values is less than or equal to a threshold delta, and that the region of skin is in a second condition when the delta between the stored impedance values and the historic impedance values or a metric based on the historic impedance values is greater than the threshold delta.

In these examples, the first condition can indicate generally healthy skin and the second condition can indicate damaged skin. Optionally, if the condition of the region of skin meets a criteria, the method can continue at step 718, where a majority of the first electrode elements are used to induce an electric field through the subject's body. For example, when the impedance comparisons indicate that the region of skin is in the first condition, the criteria is met and a second AC signal is applied to a majority of the first electrode elements and to a majority of the second electrode elements to induce an electric field through the subject's body (e.g., to administer TTFields to the subject). For example, the second AC signal can have a frequency between 100 and 500 kHz.

Based on the determined condition for the region of skin, the application of TTFields can be adjusted. For example, the current at any given electrode element positioned at the damaged skin can be reduced when applying the TTFields, or the TTFields therapy can be paused for a period of time so that the damaged skin can heal. Other suitable characteristics of the TTFields treatment can be adjusted based on the determined condition of the region of skin.

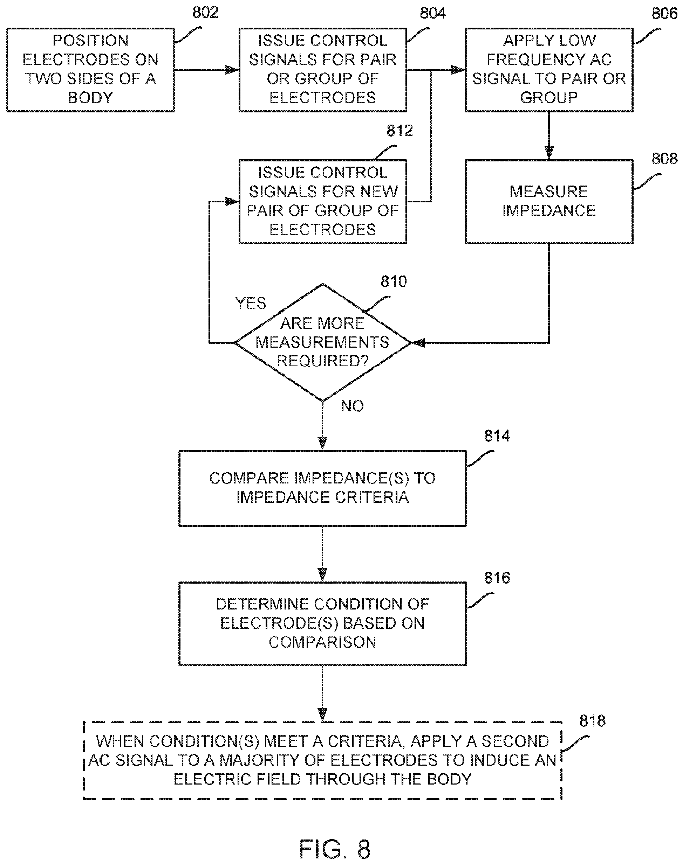

FIG. 8 is a flowchart of one example for detecting electrode element integrity using electrode elements positioned on two sides of a body. For example, two or four copies of the transducer of FIG. 3 and the system of FIG. 4A can be used to implement the flowchart of FIG. 8.

At step 802, electrode elements are positioned on two sides of a body. For example, at least four first electrode elements can be positioned on a first side of a body and at least four second electrode elements can be positioned on a second side of the body. The first electrode elements are positioned so that each of the first electrode elements is coupled to a respective region of skin on the body. Similarly, the second electrode elements are positioned so that each of the second electrode elements is coupled to a respective region of skin on the body. In some embodiments, the first electrode elements are part of an array (e.g., transducer array 50 of FIG. 3); and, in some embodiments, the second electrode elements are also part of an array.

At step 804, control signals are issued that select a subset of the first and second electrode elements. For example, a controller (e.g., controller 34 of FIG. 4A) issues control signals to configure switches (e.g., switch banks 1L and 1R of FIG. 4A) so that an electrically conductive path is formed from an AC signal generator to the selected pair or group of electrode elements. In embodiments, the subset comprises one or more of the first electrode elements and one or more of the second electrode elements.

At step 806, a low frequency AC signal is applied to the selected subset (e.g., pair) of electrode elements. For example, the AC signal can have a frequency below 20 kHz. The applied AC signal causes an electric field to be induced through the body by the first electrode elements and second electrode elements that comprise the selected subset of electrode elements. At step 808, during the application of the AC signal to the selected subset (pair), an impedance is measured and stored.

At step 810 it is determined whether more measurements are required. For example, it may be determined whether all subsets from a set of subsets of electrode elements has had an impedance measurement taken, whether any additional subsets of first electrode elements and second electrodes elements is eligible for measurement, whether a threshold number of impedance measurements have been taken, or any other suitable criteria. When more measurements are required, the flowchart progresses along a loop to step 812. When more measurements are not required, the flowchart exits the loop and progresses to step 814.

At step 812, control signals are issued that select a new subset of the electrode elements. For example, a set of electrode element subsets, each comprising at least one first electrode element and at least one second electrode element, can be traversed by the loop of the FIG. 8 flowchart until each subset in the set has been selected for impedance measurement. From step 812 the flowchart loops back to steps 806 and 808, where the AC signal is applied to the new subset of electrode elements and during this application impedance measurements are taken and stored.

The flowchart of FIG. 8 loops through steps 806, 808, 810, and 812 until more measurements are not required at step 810. Once this loop break condition is achieved, the flowchart progresses to step 814, where a condition of one or more of the electrode elements is determined based on a comparison of the stored impedances with an impedance criteria. For example, an impedance criteria can be a range of values or a threshold value, and the stored impedance values can be compared to this threshold or range. Stored impedance values can be associated with the individual electrode elements that were selected (e.g., switched on) when the measurement was taken. The condition for these individual electrode elements can be determined by comparing their associated impedance value(s) to the impedance criteria. In some embodiments, the impedances can be compared to previously stored impedance values (e.g., historic values measured over time for multiple electrode elements or historic values measured over time for an individual electrode element), or a metric calculated based on these historic values. In some embodiments, the comparing comprises comparing the measured impedances to a constant.

At step 816, a condition of electrode elements is determined based on the comparisons. For example, it can be determined that a given electrode element is in a first condition when the stored impedance values associated with the given electrode element fall within an acceptable range of the impedance criteria or meet a threshold of the impedance criteria, and that the given electrode element is in a second condition when the stored impedance values associated with the given electrode element are outside of the acceptable range of the impedance criteria or fail to meet (e.g., greater than or less than) the threshold of the impedance criteria. In another embodiment, it can be determined that the given electrode element is in a first condition when a delta between the stored impedance value(s) associated with the given electrode and historic impedance values or a metric based on the historic impedance values is less than or equal to a threshold delta, and that the given electrode element is in a second condition when the delta between the stored impedance value(s) associated with the given electrode and historic impedance values or a metric based on the historic impedance values is greater than the threshold delta.

In these examples, the first condition can indicate an operable condition for the given electrode element while the second condition can indicate a flawed condition for the given electrode element. Optionally, if the condition of the one or more electrode elements meets a criteria, the method can continue at step 818, where a majority of the electrode elements are used to induce an electric field through the body. For example, when the impedance comparisons indicate that the one or more electrode elements (e.g., a majority of the electrode elements) are in the first condition, the criteria is met and a second AC signal is applied to a majority of the first electrode elements and to a majority of the second electrode elements to induce an electric field through the body (e.g., to administer TTFields to the body). For example, the second AC signal can have a frequency between 100 and 500 kHz.

Based on the determined conditions for the electrode elements, the application of TTFields can be adjusted. For example, one or more electrode elements can be switched off so that these electrode elements do not participate in the application of TTFields. Other suitable characteristics of the TTFields treatment can be adjusted based on the determined condition of the electrode elements.

In some embodiments, based on the determined electrode condition and/or determined region of skin condition, the controller 34 controls the switches in bank 1L to either turn on or turn off the current (which originates in the AC voltage generator 35) to each of the corresponding electrode elements 52 (E1-E4) during treatment using TTFields. For example, to leave the current on for all four of the electrode elements 52, all four of the switches in bank 1L should be closed. To interrupt the current that reaches electrode element E1, switch 1 in bank 1L should be opened; and to interrupt the current that reaches electrode element E2, switch 2 in bank 1L should be opened; etc.

Controlling the current that is routed through individual electrode elements based on electrode condition can be used to reduce or eliminate the decrease in average current that is coupled into the person's body. This, in turn, can advantageously reduce or eliminate the decrease in strength of the electric field at the tumor. For example, the controller 34 can be programmed to alternately switch the current on and off for individual electrode elements during TTFields treatment without affecting the current that passes through the remaining electrode elements. In some embodiments, the controller 34 may be configured to increase the current through the remaining electrode elements in order to compensate for the reduction in current through a single electrode element during TTFields treatment.

One can differentiate between an improper set-up for the transducer arrays due to a defective electrode (such as a defect in the dielectric layer of the electrode element, or if the hydrogel beneath an electrode element dries out) as opposed to a skin defect because all impedance measurements that include the defective electrode will register as similarly falling outside of the threshold of the impedance criteria, and repositioning the array will not correct the unacceptable impedance measurements. On the other hand, an improper set-up for the transducer arrays due to a skin defect will likely show variance, especially when the array is relocated to different positions since the skin will not be defective in all locations, or at least will be variably defective.

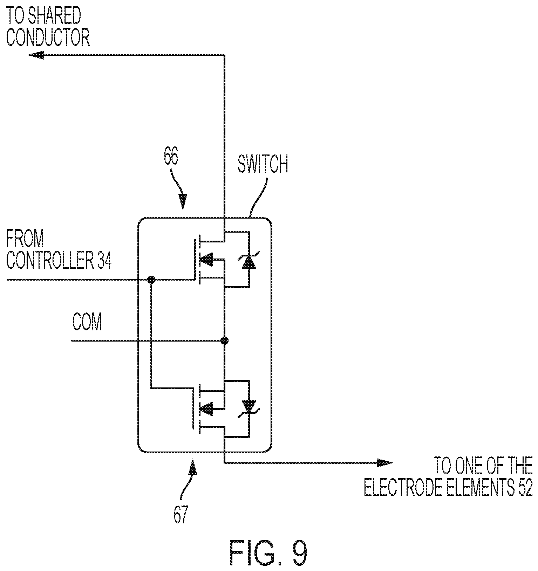

FIG. 9 is a schematic diagram of a circuit that is suitable for implementing each of the switches in the FIGS. 4A-4C embodiments described above, as well as the corresponding switches or banks of switches for the anterior and posterior channels (not shown). The circuit includes two field effect transistors 66, 67 wired in series, which is a configuration that can pass current in either direction. One example of a suitable FET for this circuit is the BSC320N20NSE. (Note that the diodes depicted in FIG. 9 are inherently included within the FETs 66, 67 themselves.) The series combination of the two FETs 66, 67 will either conduct or block the flow of electricity, depending on the state of the control input that arrives from one of the digital outputs of the controller 34 described above. When the series combination is conducting, current can flow between the shared conductor and the respective electrode element 52. On the other hand, when the series combination of FETs 66, 67 is not conducting, current will not flow between the shared conductor and the respective electrode element 52.

In the embodiments described above, the support structure 59 is configured to hold the electrode elements 52 against the subject's body so that the dielectric layer of the electrode elements 52 faces the subject's body and can be positioned in contact with the subject's body. But in alternative embodiments, the dielectric layer of each electrode element is omitted, in which case the support structure 59 holds the electrode elements 52 against the subject's body so a conductive surface of the electrode elements 52 faces the subject's body and can be positioned in contact with the subject's body. Optionally, in these embodiments, a layer of hydrogel or conductive adhesive may be disposed between the conductive surface of the electrode elements 52 and the subject's body when the transducer array 50 is placed against the subject's body.

Embodiments illustrated under any heading or in any portion of the disclosure, including the claims, may be combined with embodiments illustrated under the same or any other heading or other portion of the disclosure unless otherwise indicated herein or otherwise clearly contradicted by context.

While the present invention has been disclosed with reference to certain embodiments, numerous modifications, alterations, and changes to the described embodiments are possible without departing from the scope ofthe present invention, as defined in the appended claims. Accordingly, it is intended that the present invention not be limited to the described embodiments, but that it has the full scope defined by the language ofthe following claims.

## Claims

1. An apparatus for applying alternating current between at least four first electrode elements (52) positioned on a first side of a body and at least four second electrode elements (52) positioned on a second side of the body, the apparatus comprising an AC signal generator (35), a plurality of switches and a controller (34) configured to control the plurality of switches,
the AC signal generator (35) being configured to generate an AC output signal from a first output and a second output, **characterized by**: ,
the plurality of switches comprising at least four first switches, each of which is configured to selectively apply the first output to a respective one of the first electrode elements (52) depending on a state of a respective control signal, at least four second switches, each of which is configured to selectively apply the second output to a respective one of the second electrode elements (52) depending on a state of a respective control signal, and at least one third switch, each of which is configured to selectively apply the second output to a respective one of the first electrode elements (52) depending on a state of a respective control signal; and
the controller (34) being configured to control the plurality of first switches, the plurality of second switches and the at least one third switch so that, in a first mode, the controller (34) issues control signals that cause a majority of the first switches to apply the first output to corresponding first electrode elements (52) and that cause a majority of the second switches to apply the second output to corresponding second electrode elements (52), and, in a second mode, the controller (34) issues control signals that cause the first switches to sequentially apply the first output to respective first electrode elements (52) in turn while the at least one third switch applies the second output to at least one respective first electrode element (52), and sequentially receive impedance measurements corresponding to respective combinations of the first electrode elements (52).

2. The apparatus of claim 1, wherein, in the second mode, the controller (34) issues control signals that cause the first switches to sequentially apply the first output to respective single first electrode elements (52) in turn while the at least one third switch applies the second output to a respective single first electrode element (52), and sequentially receive impedance measurements corresponding to respective combinations of the first electrode elements (52).

3. The apparatus of claim 1, further comprising:
at least one fourth switch, each of which is configured to selectively apply the first output to a respective one of the second electrode elements (52) depending on a state of a respective control signal;
wherein the controller (34) is configured to control the plurality of first switches, the plurality of second switches and the at least one fourth switch so that, in a third mode, the controller (34) issues control signals that cause the second switches to sequentially apply the second output to respective second electrode elements (52) in turn while the at least one fourth switch applies the first output to at least one respective second electrode element (52), and sequentially receive impedance measurements corresponding to respective combinations of the second electrode elements (52).

4. The apparatus of claim 3, wherein, in the third mode, the controller (34) issues control signals that cause the second switches to sequentially apply the second output to respective single second electrode elements (52) in turn while the at least one fourth switch applies the first output to a respective single second electrode element (52), and sequentially receive impedance measurements corresponding to respective combinations of the second electrode elements (52).

5. The apparatus of claim 1, wherein, in the first mode and based on the received impedance measurements, the controller (34) is configured to issue a control signal to cause a reduction in current at one or more of the first electrode elements (52).

6. The apparatus of claim 1, wherein the AC signal generator (35) is configured to induce an electric field having a frequency below 20 kHz when the controller (34) is receiving impedance measurements.

7. The apparatus of claim 1, wherein the at least four first electrode elements (52) are provided by a first transducer array (50; 50A, 50P, 50L, 50R) positioned on the first side ofthe body, and the at least four second electrode elements (52) are provided by a second transducer array (50; 50A, 50P, 50L, 50R) positioned on the second side ofthe body.

## Patentansprüche

1. Einrichtung zum Anlegen von Wechselstrom zwischen mindestens vier ersten Elektrodenelementen (52), die auf einer ersten Seite eines Körpers positioniert sind, und mindestens vier zweiten Elektrodenelementen (52), die auf einer zweiten Seite des Körpers positioniert sind, wobei die Einrichtung einen Wechselstromsignalgenerator (35), eine Vielzahl von Schaltern und eine Steuereinheit (34) umfasst, die so konfiguriert ist, dass sie die Vielzahl von Schaltern steuert, wobei der Wechselstromsignalgenerator (35) so konfiguriert ist, dass er ein Wechselstromausgangssignal erzeugt, das von einem ersten Ausgang und einem zweiten Ausgang, **gekennzeichnet durch**:
die Vielzahl von Schaltern mindestens vier erste Schalter umfasst, von denen jeder so konfiguriert ist, dass er den ersten Ausgang selektiv an ein jeweiliges der ersten Elektrodenelemente (52) in Abhängigkeit von einem Zustand eines jeweiligen Steuersignals anlegt, mindestens vier zweite Schalter, von denen jeder so konfiguriert ist, dass er den zweiten Ausgang selektiv an ein jeweiliges der zweiten Elektrodenelemente (52) in Abhängigkeit von einem Zustand eines jeweiligen Steuersignals anlegt, und mindestens einen dritten Schalter, von denen jeder so konfiguriert ist, dass er den zweiten Ausgang selektiv an ein jeweiliges der ersten Elektrodenelemente (52) in Abhängigkeit von einem Zustand eines jeweiligen Steuersignals anlegt; und
wobei die Steuereinheit (34) so konfiguriert ist, dass sie die Vielzahl von ersten Schaltern, die Vielzahl von zweiten Schaltern und den mindestens einen dritten Schalter so steuert, dass die Steuereinheit (34) in einem ersten Modus Steuersignale ausgibt, die bewirken, dass eine Vielzahl der ersten Schalter den ersten Ausgang an entsprechende erste Elektrodenelemente (52) anlegt, und die bewirken, dass eine Vielzahl der zweiten Schalter den zweiten Ausgang an entsprechende zweite Elektrodenelemente (52) anlegt, und in einer zweiten Betriebsart gibt die Steuereinheit (34) Steuersignale aus, welche die ersten Schalter veranlassen, nacheinander den ersten Ausgang an entsprechende erste Elektrodenelemente (52) anzulegen, während der mindestens eine dritte Schalter den zweiten Ausgang an mindestens ein entsprechendes erstes Elektrodenelemente (52) anlegt, und empfängt nacheinander Impedanzmessungen, die den entsprechenden Kombinationen der ersten Elektrodenelemente (52) entsprechen.

2. Einrichtung nach Anspruch 1, wobei in der zweiten Betriebsart die Steuereinheit (34) Steuersignale ausgibt, welche die ersten Schalter veranlassen, nacheinander den ersten Ausgang an jeweilige einzelne erste Elektrodenelemente (52) anzulegen, während der mindestens eine dritte Schalter den zweiten Ausgang an ein jeweiliges einzelnes erstes Elektrodenelement (52) anlegt, und nacheinander Impedanzmessungen empfangen, die jeweiligen Kombinationen der ersten Elektrodenelemente (52) entsprechen.

3. Einrichtung nach Anspruch 1, ferner umfassend:
mindestens einen vierten Schalter, von denen jeder so konfiguriert ist, dass er den ersten Ausgang selektiv an ein entsprechendes der zweiten Elektrodenelemente (52) in Abhängigkeit von einem Zustand eines entsprechenden Steuersignals anlegt;
wobei die Steuereinheit (34) so konfiguriert ist, dass sie die Vielzahl von ersten Schaltern, die Vielzahl von zweiten Schaltern und den mindestens einen vierten Schalter so steuert, dass die Steuereinheit (34) in einer dritten Betriebsart Steuersignale ausgibt, die bewirken, dass die zweiten Schalter nacheinander den zweiten Ausgang an jeweilige zweite Elektrodenelemente (52) anlegen, während der mindestens eine vierte Schalter den ersten Ausgang an mindestens ein jeweiliges zweites Elektrodenelement (52) anlegt, und nacheinander Impedanzmessungen empfängt, die jeweiligen Kombinationen der zweiten Elektrodenelemente (52) entsprechen.

4. Einrichtung nach Anspruch 3, wobei in der dritten Betriebsart die Steuereinheit (34) Steuersignale ausgibt, die bewirken, dass die zweiten Schalter nacheinander den zweiten Ausgang an jeweilige einzelne zweite Elektrodenelemente (52) anlegen, während der mindestens eine vierte Schalter den ersten Ausgang an ein jeweiliges einzelnes zweites Elektrodenelement (52) anlegt, und nacheinander Impedanzmessungen empfangen, die den jeweiligen Kombinationen der zweiten Elektrodenelemente (52) entsprechen.

5. Einrichtung nach Anspruch 1, wobei die Steuereinheit (34) in der ersten Betriebsart und auf der Grundlage der empfangenen Impedanzmessungen so konfiguriert ist, dass sie ein Steuersignal ausgibt, um eine Reduzierung des Stroms an einem oder mehreren der ersten Elektrodenelemente (52) zu bewirken.

6. Einrichtung nach Anspruch 1, wobei der Wechselstromsignalgenerator (35) so konfiguriert ist, dass er ein elektrisches Feld mit einer Frequenz unter 20 kHz induziert, wenn die Steuereinheit (34) Impedanzmessungen empfängt.

7. Einrichtung nach Anspruch 1, wobei die mindestens vier ersten Elektrodenelemente (52) durch eine erste Wandleranordnung (50; 50A, 50P, 50L, 50R) bereitgestellt werden, die auf der ersten Seite des Körpers angeordnet ist, und die mindestens vier zweiten Elektrodenelemente (52) durch eine zweite Wandleranordnung (50; 50A, 50P, 50L, 50R) bereitgestellt werden, die auf der zweiten Seite des Körpers angeordnet ist.

## Revendications

1. Appareil destiné à l'application d'un courant alternatif entre au moins quatre premiers éléments d'électrode (52) positionnés sur un premier côté d'un corps et au moins quatre seconds éléments d'électrode (52) positionnés sur un second côté du corps, l'appareil comprenant un générateur de signaux de courant alternatif (35), une pluralité de commutateurs et un dispositif de commande (34) configuré pour commander la pluralité de commutateurs, le générateur de signaux de courant alternatif (35) étant configuré pour un signal de sortie de courant alternatif provenant d'une première terminal et d'une seconde terminal, **caractérisé par** ;
la pluralité de commutateurs comprenant au moins quatre premiers commutateurs, dont chacun est configuré pour appliquer sélectivement la première terminal à l'un respectif des premiers éléments d'électrode (52) en fonction d'un état d'un signal de commande respectif, au moins quatre deuxièmes commutateurs, dont chacun est configuré pour appliquer sélectivement la seconde terminal à l'un respectif des seconds éléments d'électrode (52) en fonction d'un état d'un signal de commande respectif, et au moins un troisième commutateur, dont chacun est configuré pour appliquer sélectivement la seconde terminal à l'un respectif des premiers éléments d'électrode (52) en fonction d'un état d'un signal de commande respectif ; et
le dispositif de commande (34) étant configuré pour commander la pluralité de premiers commutateurs, la pluralité de deuxièmes commutateurs et l'au moins un troisième commutateur de telle sorte que, dans un premier mode, le dispositif de commande (34) émet des signaux de commande qui amènent une majorité des premiers commutateurs à appliquer la première terminal aux premiers éléments d'électrode correspondants (52) et qui amènent la majorité des deuxièmes commutateurs à appliquer la seconde terminal aux seconds éléments d'électrode correspondants (52), et dans un deuxième mode, le dispositif de commande (34) émet des signaux de commande qui amènent les premiers commutateurs à appliquer séquentiellement la première terminal aux premiers éléments d'électrode respectifs (52) en retour tandis que l'au moins un troisième commutateur applique la seconde terminal à au moins un premier élément d'électrode respectif (52), et reçoivent séquentiellement des mesures d'impédance correspondant aux combinaisons respectives des premiers éléments d'électrodes (52).

2. Appareil selon la revendication 1, dans lequel, dans le deuxième mode, le dispositif de commande (34) émet des signaux de commande qui amènent les premiers commutateurs à appliquer séquentiellement la première terminal au premiers éléments d'électrode (52) individuels respectifs en retour tandis que l'au moins un troisième commutateur applique la seconde terminal à un premier élément d'électrode (52) individuel respectif, et reçoit séquentiellement des mesures d'impédance correspondant aux combinaisons respectives des premiers éléments d'électrode (52).

3. Appareil selon la revendication 1, comprenant en outre :
au moins un quatrième commutateur dont chacun est configuré pour appliquer sélectivement la première terminal à l'un respectifs des seconds éléments d'électrode (52) en fonction d'un état d'un signal de commande respectif;
dans lequel le dispositif de commande (34) est configuré pour commander la pluralité de premiers commutateurs, la pluralité de deuxièmes commutateurs et l'au moins un quatrième commutateur, de telle sorte que, dans un troisième mode, le dispositif de commande (34) émet des signaux de commande qui amènent les deuxièmes commutateurs à appliquer séquentiellement la seconde terminal aux seconds éléments d'électrode (52) respectifs en retour tandis que l'au moins un quatrième commutateur applique la première terminal à au moins un second élément d'électrode (52) respectif, et reçoit séquentiellement des mesures d'impédance correspondant à des combinaisons respectives des seconds éléments d'électrode (52).

4. Appareil selon la revendication 3, dans lequel, dans le troisième mode, le dispositif de commande (34) émet des signaux de commande qui amènent les seconds commutateurs à appliquer séquentiellement la seconde terminal au seconds éléments d'électrode (52) individuels respectifs en retour tandis que l'au moins un quatrième commutateur applique la première terminal à un second élément d'électrode (52) individuel respectif, et reçoit séquentiellement des mesures d'impédance correspondant à des combinaisons respectives des seconds éléments d'électrode (52).

5. Appareil selon la revendication 1, dans lequel, dans le premier mode et sur la base des mesures d'impédance reçues, le dispositif de commande (34) est configuré pour émettre un signal de commande pour amener une réduction dans le courant au niveau d'un ou plusieurs des premiers éléments d'électrode (52).

6. Appareil selon la revendication 1, dans lequel le générateur de signal de courant alternatif (35) est configuré pour induire un champ électrique ayant une fréquence inférieure à 20 kHz lorsque le dispositif de commande (34) reçoit des mesures d'impédance.

7. Appareil selon la revendication 1, dans lequel les au moins quatre premiers éléments d'électrode (52) sont fournis par un premier réseau de transducteurs (50 ; 50A, 50P, 50L, 50R) positionné sur le premier côté du corps, et les au moins quatre seconds éléments d'électrode (52) sont fournis par un second réseau de transducteurs (50 ; 50A, 50P, 50L, 50R) positionné sur le second côté du corps.
